# EUROPÄISCHE PATENTSCHRIFT

(11) **EP 0 451 237 B1**
(45) Veröffentlichungstag und Bekanntmachung des Hinweises auf die Patenterteilung: **06.04.1994**
(21) Anmeldenummer: 90915084.9
(22) Anmeldetag: 10.10.1990
(51) Int. Cl.: A61K 9/127, A61K 31/57

(54) **PHARMAZEUTISCHE PRÄPARATE**
PHARMACEUTICAL PREPARATIONS
PREPARATIONS PHARMACEUTIQUES

(30) Priorität: 27.10.1989 DE 3936328
(43) Veröffentlichungstag der Anmeldung: 16.10.1991
(73) Patentinhaber: SCHERING AKTIENGESELLSCHAFT, 13342 Berlin (DE)
(72) Erfinder: RÖSSLING, Georg, D-1000 Berlin 28 (DE); SACHSE, Andreas, D-1000 Berlin 10 (DE); RIEDL, Jutta, D-1000 Berlin 19 (DE)
(86) Internationale Anmeldenummer: DE9000776
(87) Internationale Veröffentlichungsnummer: WO9106284

(56) Entgegenhaltungen:
- EP-A- 0 240 630
- WO-A-86/01402
- WO-A-89/01327
- Deutsche Apotheker Zeitung, vol. 128, no. 41, Suppl. 10, 13. Oct. 1988, (Stuttgart, DE) H.F. Merk et al.: "Therapie der androgenetischen Alopezie", pages 11-15, see pages 1, 12
- Auszüge aus E. Mutschler, Arzneimittelwirkungen- Lehrbuch der Pharmakologie und Toxikologie
- M. Mezei et al. Liposoma-selective drug delivery system for the tropical route of administration: gel dosage form J.Pharm. Pharmacol. 34, 473-474, 1982.
- W. Fork, Allg. u. Spezielle Pharmakologie u. Toxikologie, 4. Aufl. Seiten 364-368, Verlag Bibliogr. Institut Mannheim/Wien/Zürich

## Beschreibung

Die Erfindung betrifft pharmazeutische Präparate, welche durch einen Gehalt an in Liposomen verkapseltem Cyproteronacetat (=21-Acetoxy-6-chlor-1,2α-methylen-4,6-pregnadien-3,20-dion), Chlormadinonacetat (=17α-Acetoxy-6-Chlor-4,6-pregnadien-3,20-dion) oder 17α-Propylmesterolon (=17β-Hydroxy-1α-methyl-17α-M-propyl-5α-androsten-3-on) gekennzeichnet sind. Diese Präparate sind vorzugsweise in Form einer Lotion, eines Gels oder einer Salbe zubereitet und dienen insbesondere zur topischen Behandlung androgenabhängiger Erkrankungen.

Es ist bekannt, daß man pharmazeutische Präparate, die antiandrogen wirksame Substanzen enthalten, zur Behandlung androgenabhängiger Erkrankungen der Frau, wie zum Beispiel der Akne, der Seborrhoe, der androgenabhängigen Alopecie und des Hirsutismus verwendet. Die in der Bundesrepublik Deutschland zu diesem Zweck verwendeten handelsüblichen Präparate enthalten als antiandrogenen Wirkstoff Cyproteronacetat oder Chlormadinonacetat und werden oral appliziert.

Wegen ihrer antiandrogenen Wirksamkeit dürfen derartige Präparate nicht zur Behandlung während der Schwangerschaft oder zur Behandlung von Männern, die an androgenabhängigen Erkrankungen, wie der Seborrhoe oder androgenabhängiger Alopecie leiden, verwendet werden. In Hinblick auf diese Beschränkung der Anwendbarkeit und die Nebenwirkungen der oral applizierten Präparate besteht ein Bedarf antiandrogene Wirkstoffe enthaltende Präparate bereitzustellen, die bei topischer Applikation eine gute Wirksamkeit entfalten.

Bislang durchgeführte Untersuchungen gut verträglicher, topisch applizierbare Präparate zu entwickeln, welche die obengenannten Wirkstoffe enthalten, verliefen bislang unbefriedigend, da die Wirkstoffe in den bislang untersuchten galenischen Formulierungen eine zu geringe Penetrationsgeschwindigkeit durch die Haut besitzen (WO-A 86 01402 und Deutsche Apotheker Zeitung 128, 1988, Nr. 41, p11-15).

Die WO-A-8901 327 beschreibt Präparate auf Liposomenbasis, welche Pregnenolon enthalten. Diese Zubereitungen sollen eine widerbelebende, aufbauende bzw. verjüngende Wirkung auf die Haut ausüben.

Es wurde nun gefunden, daß man überraschenderweise eine therapeutisch ausreichende und gleichmäßige Penetrationsgeschwindigkeit diese antiandrogenen Wirkstoffe durch die Haut erzielt, wenn man dieselben in Liposomen verkapselt. So ist es möglich topisch applizierbare Präparate bereitzustellen, die ihre Wirkung im wesentlichen an den peripheren Androgenrezeptoren im Applikationsbereich entfalten. Systemische Nebenwirkungen werden hierdurch vermieden oder minimiert. Dadurch daß der Wirkstoff in den Liposomen konzentriert ist, ist es möglich, geringe Mengen an Wirkstoff zu verwenden und trotzdem eine hohe Wirkstoffkonzentration am Wirkort zu erzielen. Erwähnenswert ist auch, daß die liposomal verkapselten antiandrogenen Wirkstoffe über einen längeren Zeitraum abgegeben werden (substained release).

Zur Verkapselung der Wirkstoffe in Liposomen können Verfahren verwendet werden, die dem Fachmann an sich wohl bekannt sind. So kann man beispielsweise die antiandrogenen Wirkstoffe und Liposomen bildende Substanzen in einem organischen Lösungsmittel lösen, die Lösung in eine wässrige Phase eintragen und gegebenenfalls nach Homogenisierung das Lösungsmittel destillativ entfernen. Üblicherweise werden die 5 bis 100 fache Gewichtsmenge Liposomen bildende Substanz pro g Wirkstoff verwendet.

Geeignete Liposomen bildende Substanzen sind insbesondere Phospholipide, wie die Sphingomyeline, die Plasmalogene, die Phosphatidylcholine, die Phosphatidylethanolamine, die Phosphatidylserine, die Phosphatidylinosite und die Cardiolipine oder auch Gemische dieser Lipide (Dr. Otto-Albert Neumüller: Römpps Chemie-Lexikon; Franck'sche Verlagshandlung, Stuttgart(DE) 2665, 3159, 3920 und 4045) und Gemische dieser Phospholipide mit Cholesterin und/oder Ladungsträgern wie zum Beispiel Stearylamin, Stearinsäure oder Dicetylphosphat.

20 Gewichtsprozent Phospholipid oder Gemisch bezogen auf die die wässrige Phase verwendet. Geeignete Gemische enthalten etwa bis zu 60 Gewichtsprozent Cholesterin und bis zu 15 Gewichtsprozent Ladungsträger. Als Lösungsmittel für die Phospholipide oder Gemische und Wirkstoffe verwendet man vorzugsweise Methanol, Ethanol, Isopropanol, Diethylether, Aceton, Chloroform und Gemische dieser Lösungsmittel.

Da die Lipide oxidationsempfindlich sind, wird das Verfahren zweckmäßigerweise unter einer Inertgasatmosphäre, wie Stickstoff oder Argon durchgeführt und die erhaltenen wässrigen Liposomenlösung durch Zugabe von Antioxidantien, wie Natriumascorbat, Tocopherol oder Natriumhydrogensulfit stabilisiert.

Ferner können die wässrigen Liposomenlösungen noch zusätzliche Hilfsstoffe, wie Bactericide, Konservierungsmittel, Puffersubstanzen oder auch Wirkstoffe enthalten, um Kombinationspräparate herzustellen. Derartige Kombinationspräparate sind beispielsweise solche, die zusätzlich noch Kortikoide (wie zum Beispiel Hydrocortison, Fluocortzolon, Diflucortolon, Methylprednisolon und deren Ester wie das Methylprednisolonaceponat) enthalten.

Die Verkapselung der antiandrogenen Wirkstoffe in Liposomen kann unter den gleichen Bedingungen durchgeführt werden, wie die vorbekannten Verfahren dieser Art (Pharmazie in unserer Zeit 11, 1982, 97-108, Pure Appl. Chem., 53, 1981, 2241-2254). Das Verfahren zur Verkapselung der antiandrogenen Wirkstoffe eignet sich sowohl zur Herstellung multilamellarer Liposomen als auch zur Herstellung unilamellarer Liposomen.

Bei dem erfindungsgemäßen Verfahren ist es aber andererseits auch möglich, das Lösungsmittel nicht durch Destillation sondern mittels der bekannten Verfahren der transmembranen Destillation (Chem. Ing. Techn. 56, 1984, 514-521; J. of Membrane Sci., 39, 1988, 25-42; DE-A 33 12 359) und Pervaporation (Swiss Chem. 10, 1988, 45-51; ACS Symposium 281, 1985, 467-478; Chem. Ing. Tech. 60, 1988, 590-603) zu entfernen.

Die so dargestellten wirkstoffhaltigen Liposomensuspensionen können bei Bedarf mittels Wasser verdünnt und/oder mit Verdickungsmitteln, wie Hydroxyethylzellulose, Methylzellulose, Aerosil® (Hersteller Degussa AG, DE-6000 Frankfurt) Carbopol® (B.F. Goodrich Chem., USA 44131 Cleveland/Ohio) etc. versetzt werden um so streichfahige Gele herzustellen.

Andererseits ist es aber auch beispielsweise möglich, die Suspensionen mittels Gefriertrocknung zur Trockne einzuengen und den erhaltenen Rückstand in eine Salbengrundlage oder eine Creme einzuarbeiten.

Die optimale Wirkstoffkonzentration in den fertigen pharmazeutischen Präparaten ist von der Art des Wirkstoffs und der galenischen Zubereitung abhängig und muß im Einzelfalle mittels der üblichen Vorversuche ermittelt werden. In der Regel wird es ausreichend sein, wenn man pharmazeutische Präparate anwendet, die 0,001 bis 1 mg und vorzugsweise 0,01 bis 0,5 mg antiandrogenen Wirkstoff pro Gramm des Präparats verwendet.

Die nachfolgenden Ausführungsbeispiele dienen zur näheren Erläuterung der Erfindung.

### Beispiel 1

(Filmmethode mit anschließender Hochdruck-Homogenisation.)

2,0 g PC S 100 (Hersteller Lipoid KG, DE-Ludwigshafen), 0,2 g Cholesterin und 50 mg 17α-Propylmesterolon werden in 100 ml 95 %igem Ethanol gelöst. Dann wird die Lösung in einem 500 ml Rundkolben am Rotationsverdampfer zur Trockne eingeengt, wobei sich ein Lipidfilm an der Glaswand ausbildet. Dieser Lipidfilm wird mit 100 ml bidestilliertem Wasser abgelöst. Anschließend wird die erhaltene Liposomensuspension mit einem Hochdruckhomogenisator (Microfluidizer®, der Firma Microfluid, Corp., USA) bei 400 MPa und 25° C homogenisiert und durch ein Filter von 0,2µm filtriert.

Die erhaltene Liposomensuspension enthält Liposomen mit einer mittleren Größe von 93 nm. Der Gehalt an Phosphatidylcholin liegt bei 19,2 mg/ml, der Gehalt an verkapseltem Wirkstoff beträgt 0,46 mg/ml.

### Beispiel 2

(Eine modifizierte reversed-phase-evaporation Methode.)

2,5 g PC E 100 (Hersteller Lipoid KG, DE-Ludwigshafen) und 0,2 g Cyproteronacetat werden in 100 ml Diethylether gelöst. Dazu gibt man 50 ml 0,015 M wässrigen Tris-HCl Puffer (pH 7,4) und homogenisiert das Zweiphasengemisch im Hochdruckhomogenisator (Microfluidizer®, der Firma Mikrofluid Corp., USA) bei 20 MPa und 25° C. Die entstandene Emulsion wird am Rotationsverdampfer bei 4000 Pa und 23° C vom Diethylether befreit und durch ein Filter von 0,45µm filtriert.

Die erhaltene Liposomensuspension enthält Liposomen mit einer mittleren Größe von 250 nm. Der Gehalt an Phosphatidylcholin liegt bei 48 mg/ml, der Gehalt an verkapseltem Wirkstoff beträgt 1,1 mg/ml.

### Beispiel 3 und 4

(Herstellung eines liposomalen Hydrogels)

Die gemäß Beispiel 1 und 2 hergestellten Liposomen-Suspensionen werden mit 0,18 % p Hydroxybenzylmethylester und 0,02 p Hydroxybenzylpropylester und 2 % Hydroxyethylzellulose versetzt und 5 Minuten mit 600 Umdrehungen pro Minute und dann kurzfristig mit 3000 Umdrehungen pro Minute gerührt. Dann läßt man die Mischungen 24 Stunden ruhen.

Die Zubereitungen haben eine mittelweiche Konsistenz, der Viskositätswert liegt bei etwa 30 000 m Pa s. In der Zubereitung sind die Liposomen weiterhin in der wässrigen Phase suspendiert. Sie sind intakt, durch das bikohaerente System aber mechanisch immobilisiert und somit vorteilhaft voneinander getrennt.

### Beispiel 5 und 6

(Herstellung eines liposomalen Lipogels)

Die gemäß Beispiel 1 und 2 hergestellten Liposmen-Suspensionen werden gefriergetrocknet. Der getrocknete Liposomenkuchen wird mit einer Schlagzeugmühle zerkleinert und das entstandene Pulver portionsweise mit soviel einer Salbengrundlage verrieben, die aus Vaseline besteht, welche 0,02 % 2,6-Di-tert.-butyl-4-methylphenol (=BHT) als Antioxidans enthält, daß die Wirkstoffkonzentration in der fertigen Salbe bei 0,5 mg/g liegt.

### Beispiel 7

(Herstellung eines Kombinationspräparates)

2,0 g PC S 100 (Hersteller Lipoid KG, DE-Ludwigshafen), 0,2 g Cholesterin und 50 mg 17α-Propylmesterolon und 25 mg Methylprednisolonaceponat werden in 100 ml 95 %igem Ethanol gelöst. Dann wird die Lösung in einem 500 ml Rundkolben am Rotationsverdampfer zur Trockne eingeengt, wobei sich ein Lipidfilm an der Glaswand ausbildet. Dieser Lipidfilm wird mit 100 ml bidestilliertem Wasser abgelöst. Anschließend wird die erhaltene Liposomensuspension mit einem Hochdruckhomogenisator (Microfluidizer®, der Firma Microfluid, Corp., USA) bei 400 MPa und 25° C homogenisiert und durch ein Filter von 0.2µm filtriert.

Die erhaltene Liposomensuspension enthält Liposomen mit einer mittleren Größe von 93 nm. Der Gehalt an Phosphatidylcholin liegt bei 19,2 mg/ml, der Gehalt an verkapseltem Wirkstoff beträgt 0,46 mg/ml.

## Patentansprüche

1. Pharmazeutische Präparate gekennzeichnet durch einen Gehalt an in Liposomen verkapseltem Cyproteronacetat, Chlormadinonacetat oder 17α-Propylmesterolon.

2. Pharmazeutische Präparate gemäß Patentanspruch 1 in Form einer Lotion, eines Gels oder einer Salbe.

3. Pharmazeutische Präparate gemäß Patentanspruch 1 und 2 zur topischen Behandlung androgenabhängiger Erkrankungen.

4. Pharmazeutische Präparate gemäß Patentanspruch 1 bis 3, dadurch gekennzeichnet, daß sie als Wirkstoffe zusätzlich noch in Liposomen verkapselte Kortikoide enthalten.

## Claims

1. Pharmaceutical preparations characterised in that they contain, encapsulated in liposomes, cyproterone acetate, chloromadinone acetate or 17α-propylmesterolone.

2. Pharmaceutical preparations according to patent claim 1 in the form of a lotion, a gel or an ointment.

3. Pharmaceutical preparations according to patent claims 1 and 2 for the topical treatment of androgen-dependent disorders.

4. Pharmaceutical preparations according to patent claims 1 to 3, characterised in that they contain in addition, as active ingredients, corticoids encapsulated in liposomes.

## Revendications

1. Préparations pharmaceutiques caractérisées en ce qu'elles contiennent de l'acétate de cyprotéron, de l'acétate de chlormadinon ou du 17α-propylmestérolon, encapsulé dans des liposomes.

2. Préparations pharmaceutiques selon la revendication 1 en lotions, gels ou pommades.

3. Préparations pharmaceutiques selon les revendications 1 et 2 pour le traitement local de maladies androgénodépendantes.

4. Préparations pharmaceutiques selon les revendications 1 à 3, caractérisées en ce qu'elles contiennent en outre comme matières actives des corticoïdes encapsulés dans les liposomes.
